## Europäisches Patentamt
## European Patent Office
## Office européen des brevets

(19)

(11) Publication number: **0 009 691**
**A1**

(12)

# EUROPEAN PATENT APPLICATION

(21) Application number: **79103450.7**

(22) Date of filing: **17.09.79**

(51) Int. Cl.³: **A 61 K 7/047**

(30) Priority: **18.09.78 US 943055**

(43) Date of publication of application:
**16.04.80 Bulletin 80/8**

(84) Designated Contracting States:
**BE CH DE FR GB IT LU NL SE**

(71) Applicant: **Spray Nail, Inc.**
**P.O. Box 40335**
**Indianapolis Indiana 46240(US)**

(72) Inventor: **Mullin, Robert T.**
**135 Raintree Drive**
**Zionsville, In. 46077(US)**

(72) Inventor: **Harris, Joseph M.**
**800 E. Washington**
**Muncie, In. 47302(US)**

(74) Representative: **Speidel, Eberhardt**
**Postfach 1320**
**D-8035 Gauting(DE)**

(54) **Lacquer remover.**

(57) A remover, such as for removing nail polish, lacquer, or enamel, in which the remover preparation is provided for application in the form of a foam which provides handiness, convenience, and other advantages. Other forms are presented also.

EP 0 009 691 A1

- 1 -

## LACQUER REMOVER

This invention relates to a removal preparation and application procedure, and it is particularly desirable as for removal of so-called nail polish or nail lacquer or nail enamel from a person's fingernails or toenails.

Concepts of the present invention provide, in a desirable embodiment, a lacquer-remover preparation which is packaged in a dispenser, with the remover substance dispensed therefrom and applied in the form of a foam. Other forms of the remover preparation, with other types of containers and dispensing means, are also included.

The concepts also provide an advantageous and desirable remover substance or mixture.

Many advantages are provided by the inventive concepts. Particularly contrasting conventional or usual removal methods for removing nail polish or lacquer from fingernails, for example, the present invention overcomes or greatly reduces the messiness, bother, and other disadvantages of conventional liquid polish removers.

That is, conventional nail polish remover liquid is somewhat messy to apply and use, usually being done with the aid of cotton swabs or tissues. If the liquid spills, either some drops or the whole bottle, it has a very bad

- 2 -

and difficult-to-repair effect on the finish of many surfaces.  Its odor is often not pleasant.  The polish tends to smear onto skin regions adjacent the nail.  The physiological effect of conventional removal liquid is a harsh or caustic drying effect on the user's nails and skin, causing a disadvantageous removal of natural oils from the user's skin.  Conventional remover can be painful if the user has any hangnail or cut or scratch on the cuticle or other skin adjacent the nail itself.  Much of the remover liquid is wasted, by either spillage or evaporation. Extra time and bother is often used, to avoid spillage and evaporation, by a succession of closure and opening procedures for the remover liquid container's cap.  It is a fire hazard.

The concepts of the present invention, quite in contrast, provide in a preferred embodiment a convenient ease of application and procedure, whereby a dispensing of a foam-type remover is easily done specifically onto the nail and only a small area adjacent the nail.  It need be left on only several seconds, much less than a minute, and usually about only one-fourth of a minute.  Then a relatively simple wipe-off with a tissue is usually all that is required; and the nail is not only satisfactorily clean of the polish, but neither the nail nor the adjacent skin is apparently harmed in the least, and the nail has a nice odor and feel.

Further, there is only a minimal danger of any spilling onto a surface which would be damaged, and there is no unpleasant odor; and any fire hazard of the lacquer solvent is minimal, much less than with conventional removers.  Moreover, the succession of closing and opening of a bottle-cap is avoided.

- 3 -

Further, the foam nature seems. to provide a special advantage, in that as the foam bubbles break down on the user's nail, there is achieved a sort of an automatically-achieved time-delay action by which more of the solvent will be successively fed to the nail lacquer, achieving a more thorough lacquer-removal per unit of solvent dispensed.

In considering the nature and the many advantageous of the present invention, those factors may be. possibly easier evaluated if one might look by hindsight to the disadvantageous but long-standing and long-unimproved prior art, particularly since the problem of nail polish removal has so long existed, without much change of procedure or nature thereof or of the removal substance itself. And after all, the acetone-base removal liquids have been known for many years; and also, foamy dispensate and foam-dispensers have been known for many years for such things as shaving cream, depillatories, upholstery cleaners, etc.

Perhaps the answer to such a hindsight inquiry is in either one or more of certain factors, or perhaps a combination of them. That is, perhaps the failure of the prior art has been just a non-creativeness, a lack of the creative insight to create a substantial change in a product which has existed for scores of years in a form and nature which, after all, is of course workable and useful even though it has had the known disadvantages such as mentioned above. Or, perhaps attempts of improvement in the prior art have been toward other characteristics or other factors of the polish remover substance or procedure; and the present inventive concepts, particularly with respect to the foam-type embodiment, are a departure from the prior art product and from the prior art's attempted changes.

- 4 -

According to concepts of the present invention or discovery, the polish remover in a preferred embodiment is provided as a foamy dispensate, greatly facilitating the application thereof and providing other advantages already mentioned.

Several embodiments have been found to be desirable and workable, these showing percentages on a weight/weight basis:

A preferred embodiment "A":

| | |
|---|---|
| Glycerin | 10.10 |
| Stearic acid | 7.14 |
| KOH | 0.82 |
| Water | 18.37 |
| Alcohol (SDA-40) | 15.31 |
| Acetone | 30.82 |
| Ethyl acetate | 17.35 |

The percentages throughout do not count in other substances which may or may be present, such as propellant, fragrance, etc.

Certain things should be noted as to this embodiment, as well as to the others. Very little KOH is required. Although it is expected to form a salt with stearic acid, that is, potassium stearate (a "soap") its general effect is that of a catalyst. Thus the KOH and stearic acid create a foaming means or agent. Also, the water and the alcohol serve generally the same purpose of a carrier, although the alcohol has an extra effect of its higher volatility hastening a drying process, and the alcohol is a mutual solvent to the propellant and the foaming agent and the emollient. Thus if one of these ingredients is

- 5 -

increased, the other may be correspondingly decreased, except that some alcohol is desirably included to provide its special properties noted above. Similarly, the acetone and the ethyl acetate both serve a solvent function; however, other solvents such as other ketones (e.g., methyl isobutyl ketone, MIBK) or other acetates may be used. Thus one may be increased, with a corresponding decrease of the other. The glycerin is an emollient or conditioner; and the other emollients (such as lanolin, peanut oil, etc.) may be used instead of, or with, the glycerin shown above.

Also, in dispensers for foamy dispensate application, desirably the above mixture of "concentrate" is provided as 90%, the other 10% being a propellant known as A-46, containing about 87% iso-butane and about 13% propane.

Further, it is to be noted that whatever foaming agent is used, it must be compatible with the solvent; and preferably, just enough foaming agent is used to get the substance to foam, because of extra cost of certain foaming agents and because it is desired that the foam break down rapidly to achieve a fast contacting of the solvent with all of the area having polish or lacquer to be removed. Although this foaming agent of this embodiment is the stearic acid and KOH, it is believed that others of the prior art would be also operable, such as metallic salts of fatty acids (e.g., calcium stearate), salts of complex organic phosphorus esters (e.g., GAFAC LO-529 and GAFAMIDE CDD-518, sold by General Analine and Film Corp., 140 W. 51st Street, New York, N.Y. 10020), sulfates, sulfonates, metallic and non-metallic salts, and others known to the prior art.

As a comment generally applicable to any embodiment in which acetone is mentioned as a solvent, other ketones or solvents (e.g., methyl ethyl ketone, methyl iso-buthyl ketone, methyl acetate, iso-propyl acetate, butyl acetate) may be used as an alternative to the acetone.

As shown in this embodiment, the solvent preferably includes an ester (the ethyl acetate listed is an ester) providing a reduced flammability and more desirable scent in comparison to acetone itself, and also serves as an emollient or conditioner.

If such alternatives are used, probably an increase of the stearic acid and the KOH will be desirable, depending upon what is needed to reate the desired foam characteristics in consideration of the particular solvent alternative used.

Also as applicable to the various embodiments, whereas here the carrier is shown as water and alcohol, other carriers can presumably be used such as polyvinylpyrrolidone and/or fatty alcohols (e.g., cetyl alcohol, oleyl alcohol, and stearyl alcohol).

The glycerin serves as a skin conditioner and emollient. Other emollients could be used, such as lanolin, mineral oil, fatty esters, and glycols. Polyhydric alcohols (e.g., sorbitol, fatty esters, and fatty acid amino (or amino) betaine derivatives illustrate other conditioners which could be used.

Continuing the comments generally applicable to all the embodiments, it should be noted that there seems to be no close range or limits as to the various ingredients. That is, for exemple, in this Embodiment "A", the ranges can probably be at least as wide as follows, particularly also recalling the above comments concerning corresponding increases and decreased of certain ingredients:

| Glycerin | about 1 to 12% |
|---|---|
| Stearic acid | about 5 to 8% |
| KOH | about 0.5 to 1.5% |
| Water | about 0 to 25% |
| Alcohol &SDA-40) | about 1 to 20% |
| Acetone | about 0 to 50% |
| Ethyl acetate | about 0 to 50% |

Embodiment "B":

This, and subsequent embodiments, are stated with actual proportions (weight/weight), as well as overall ranges (bearing in mind the above comments as to overall ranges):

| | Actual: | Range: |
|---|---|---|
| Glycerin | 10% | about 1 to 12% |
| Stearic acid | 7% | about 5 to 8% |
| KOH | 0.8% | about 0.5 to 1.5% |
| Water | 20% | about 0 to 25% |
| Alcohol (SDA-40) | 15% | about 1 to 20% |
| Acetone | 47.2% | about 40 60 60% |

As before, the above "concentrate" (that is, the composition which includes the substances just listed) is desirably about 90% (weight/weight) of a foam-dispensable mixture in which the propellant is a foaming agent, such as "A-46," formed of 87% iso-butane and 13% propane.

Embodiment "C":

In the remaining embodiments, for brevity as before the actual proportion is given, with the ranges given also in the same listing:

|  | Actual: | Range: |
|---|---|---|
| Ethyl acetate | 41.24% | about 0 to 45% |
| Methyl "Cellosolve" | 13.40% | about 0 to 35% |
| IPA | 10.31% | about 5 to 15% |
| TEA "Carbopol 941"(0.2% wt.) | 5.15% | about 2 to 10% |
| Acetone | 19.59% | about 0 to 50% |
| Calcium stearate (55% water solution, by weight) | 10.31% | about 5 to 15% |

Embodiment "D":

|  | Actual: | Range: |
|---|---|---|
| Ethyl acetate | 48.45% | about 0 to 45% |
| Methyl "Cellosolve" | 15.46% | about 0 to 35% |
| Acetone | 10.31% | about 0 to 50% |
| TEA "Carbopol 941" (0.2% wt.) | 5.15% | about 2 to 10% |
| Calcium stearate (55% water solution, by weight) | 20.62% | about 10 to 25% |

Embodiment "E":

|  | Actual: | Range: |
|---|---|---|
| Ethyl acetate | 48.45% | about 0 to 45% |
| Methyl "Cellosolve" | 15.46% | about 0 to 35% |
| Acetone | 20.62% | about 0 to 35% |
| TEA Carbopol 941" (0.2% wt.) | 5.15% | about 2 to 10% |
| Calcim stearate (55% water solution, by weight) | 10.31% | about 5 to 15% |

It will be noted that the "concentrates" shown as Embodiments "C," "D," and "E" would desirably be packed in a foam dispenser in a proportion of about 90%, the propellant making up the other 10%.

The symbols, as used herein, have significances as follow:

- 9 -

"IPA":               Iso-propyl alcohol or 2-Propanol $(CH_3)_2CHOH$ b.p. 82.4°C TOC flash point (f.p.) 59.°F. It serves as a solvent and a carrier.

"Cellosolve":      T.M. by Union Carbide Corp., for mono and dialkyl ethers of ethylene glycol and their derivatives, e.g., Methyl "Cellosolve" $CH_3OCH_2CH_2OH$ b.p. 124.5°C OC f.p. 110°F.

TEA "Carbopol":    TEA=triethanolamine $(HOCH_2CH_2)_3N$ b.p. 335°C OC f.p. 375°F

The "Cellosolve" is used as a solvent; and, similar to the comment as to solvents in the earlier embodiments, it and the other solvents may be interchanged.

The "Carbopol" refers to a resin stated as being Carboxy polymethylene or Carboxy vinyl polymer (MERCK), having the formula $CH_2=CH-\underset{x}{\phantom{-}}C$ -OH, a trademark and sold by B.F. Goodrich Chemical Co., 6100 Oak Tree Blvd., Cleveland, Ohio 44131. (Type 941: m.w. 1,000,000; Type 934: m.w. 3,000,000). Thus, "TEA Carbopol 941 (0.2% by weight)" refers to an aqueous solution of "Carbopol 941," partially neutralized by TEA, which was added in approximately the same molecular weight ratio as that of the "Carbopol" itself. It serves as a surface-active agent (surfactant), a carrier, and as an emollient.

The calcium stearate is a "soap," and that herein used was called Petrac 1255, of Petrochemicals, Inc., Ft. Worth, Texas. It serves as a foaming agent and a surfactant.

Embodiment "F":

Another embodiment, which has a low odor and is an effective polish remover, although desirably dispensed in a form of liquid or spray, or a gel, in contrast to a foam, is as follows:

| | |
|---|---|
| Acetone | 25% |
| Methyl ethyl ketone | 50% |
| Water | 20% |
| "Carbopol 934" | less than 1% |
| GAFAC RE -610 | 2.5% |
| GAFAMIDE CDD-518 | 2.5% |

Perfume may be added if desired, in this as well as other embodiments.

If this is desired as a gel or paste, the "Carbopol 934" should be increased to about 1%, and neutralized with a neutralizing agent such as TEA.

The acetone and the methyl ethyl ketone proportions, they both being solvents, may be varied correspondingly as desired, although a larger proportion of the methyl ethyl ketone is desired ro raise the flash point and thus minimize the fire hazard.

The "Carbopol 934" is a water-soluble resin generally similar to the TEA "Carbopol 941" mentioned above but with a higher molecular weight.

As to the GAFAC RE-610 and the GAFAMIDE CDD-518: The GAFAC RE-610 is a complex organic phosphate ester (an anionic surfactant) sold by General Analine and Film Corp., 140 W. 51st Street, New York, NY 10020. The GAFAMIDE CDD-518 (a non-ionic surfactant) is a coconut oil-diethanolamine condensate, CTFA name "cocamide DEA," sold by the same company. Being surfactants, these are foaming agents.

The propellant of this as a spray-dispensate is desirably a hydrocarbon propellant, such as "A-46" formed of 87% isobutane and 13% propane.

Embodiments "G," "H," and "I":

Liquid forms, such as for pump-type dispensers or self-pressurized dispensers or other liquid-application procedures, including not only liquid as such but sprays or foams or gels or pastes, have been discovered as follows:

|  | Embod. "G" | Embod. "H" | Embod. "I" |
|---|---|---|---|
| Ethylene glycol monomethyl ether, $CH_3OCH_2CH_2OH$ (Methyl "Cellosolve") | 73% |  | 36.5% |
| Ethyl acetate, $CH_3COOC_2H_5$ |  | 73% | 36.5% |
| N-methyl-2-pyrrolidone, $H_3CNCH_2CH_2CH_2CO$ (e.g. "M-Pyrol" from General Analine and Film Corp.) | 7.3% | 7.3% | 7.3% |
| Calcium stearate-(55% water solution, by weight) | 3.6% | 3.6% | 3.6% |
| Coco amino betaine (Lonzaine[R] 12C) | 0.7% | 0.7% | 0.7% |
| POE(20) sorbitan monostearate (polysorbate 60) | 0.7% | 0.7% | 0.7% |

0009691

- 12 -

|  | Embod. "G" | Embod. "H" | Embod. "I" |
|---|---|---|---|
| Carboxy polymethylene | 14.6% | 14.6% | 14.6% |
| Fragrance | q.v. | q.v. | q.v. |

Advantages of these forms include a reduction of the fire hazard, reduction of the toxicity, and reduction of odor, yet they still maintain effectiveness of the polish remover characteristics.

Either the methyl "Cellosolve" ingredient or the ethyl acetate may be used wholly or partially in place of the other in these embodiments, both being solvents; and this is the difference indicated between embodiments "G," "H," and "I". Other variations in their relative proportions are assumed also to be workable and useful in a formulation for this purpose.

The calcium stearate, the coco amino betaine, and the sorbitan ingredients may be varied, presumably as high as about 5% for any of them, leaving out the others partially or wholly so that the total weight proportion of all three would be only about 5%. They serve as foam agents, are surfactants, and also act as emulsifiers and lubricants. Adjustments as to concentration vary according to the desired means of dispensing, i.e., as a spray or foam or liquid or gel.

The coco amino betaine used was Lonzaine 12 C, 32% active material, as sold by Lonza, Inc., No. 22-10 Route 208, Fair Lawn, N.J., 07410. This material is an amphoteric surfactant. It promotes foam properties.

The POE refers to polyoxyethylene; it is reacted with sorbitan monostearate, and forms a non-ionic surfactant that functions as a solubilizing agent for the perfume addition. The form used was that of "Tween 60", which is a material sold under that trademark by ICI United States, its Atlas Chemicals Div., New Murphy Road and Concord Pike, Wilmington, Delaware, 19897.

The carboxy polymethylene (TEA "Carbopol 934") used was 0.2% (by weight) water solution which was neutralized by the TEA reagent. As a triethanolamine salt gel it functions as a thickener, stabilizer, and flow control agent; and it is an anionic surfactant. Probably, up to a 20% upper limit, the amount of this ingredient may be raised if increase in thickness of suspension is desired, this depending upon what propellant, if any, is to be used. Conversely, if less viscosity is desired, less of this reagent should be used, even none at all if the composition is to be dispensed in free-flowing liquid form.

The fragrance reagent used was that of "Lemon No. 498585" sold by Universal Flavor and Fragrance Co., 5650 W. Raymond Street, Indianapolis, Indiana, 46241, which is a special lemon fragrance which effectively masks the odor of the ethylacetate.

As to the N-methyl-2-pyrrolidone, it advantageously raises the flash point, reducing volatility in contrast to that of ethyl acetate, yet it serves as a solvent for the polish or lacquer material being removed. Having properties of a solvent, it may be used in the other embodiments also.

It is thus seen that a lacquer remover preparation, particularly in a foam-type application, according to the present inventive concepts, provides a desired and advantageous substance for the removal of lacquer or nail polish, or nail enamel, achieving many advantages such as ease, convenience, and handiness, and other advantages of less harm to the person's nails and skin, less hazard from spillage and surface-marring, less fire hazard, improved odor, less loss by spillage or evaporation, etc. Even without the foam-nature of the preparation, most of these advantages are still present.

Accordingly, it will thus be seen from the foregoing description of the invention according to these illustrative embodiments, that the present invention or discovery provides new and useful concepts of lacquer removal preparation and procedure, yielding desired advantages and characteristics, and thus accomplishing the intended objects, including those hereinbefore pointed out and others which are inherent in the invention.

Modifications and variations may be effected without departing from the scope of the novel concepts of the invention; accordingly, the invention is not limited to the specific embodiments or forms or formulations herein described or shown.

For example, the inventive concepts include herein embodiments for polish removal substances which are not in the form of a foam even though the foam nature seems to provide special advantages. Thus, for example, the composition shown for Embodiment "F" was noted as preferably in a form of a liquid or spray or gel in contrast to a foam.

The other embodiments "F" through "I" may be modified as desired with respect to form; for example, they may be provided with additional foaming agents, and with a propellant or other pressure systems. Perfume or other fragrances may be incorporated as desired. Further, ingredients of one embodiment may be utilized in other embodiments also, to the extent that they perform their desired function, i.e., solvents for solvents, foams for foams, emollients for emollients, etc.

Although the utilization is herein mentioned primarily as that for removal of nail polish or lacquer, the inventive concepts include the use of these compositions for whatever purpose may be achieved, particularly removal of other lacquer coatings. Thus, as used herein, the term "nail polish" is used to indicate also whatever is the lacquer or enamel which is to be removed by the preparation.

C L A I M S

1. A remover preparation for removing nail polish, in which
the remover preparation is dispensed in a foam or foam-
like nature, and in which the preparation remains stable
in its foam or foam-like condition throughout a time-
delayed breakdown of the foam or foam-like condition to
a liquid, thereby allowing solvent penetration into the
nail polish over an extended period of time, to thereby
achieve its polish-solubility and effective polish
removal, with only a small amount of liquification of
the preparation during its time-delayed breakdown,
feeding in a successive manner small increments of the
solvent in the then-liquid form to operatively contact
the nail polish.

2. The invention as set forth in Claim 1, in which the
preparation contains a foaming agent or agents which
provide the aforesaid time-delay breakdown of the foam
or foam-like condition of the preparation while it is on
the user's nail.

3. The invention as set forth in either of Claims 1 or 2,
in which the foaming agent or agents, which achieve the
dispensing to be in a foam or foam-like nature, is/are
selected from a group consisting of stearic acid, soaps
or metallic salts of fatty acids, calcium stearate,
complex organic phosphate ester, salts of complex organic
phosphorus esters, coconut diethanolamide or coconut oil-
diethanolamine condensate, sulfates, sulfonates, metallic
and non-metallic salts, surfactants and amphoteric
surfactants or coco amino betaine, carboxy polymethylene
resin or carboxy vinyl polymer resin partially
neutralized by triethanolamine, polyoxyethylene sorbitan monostearate, and the amount of such foaming agent or agents is significantly large enough so as to achieve the aforesaid time-delay effect.

4. The invention as set forth in Claim 3, in which the preparation contains also KOH as a catalyst for the stearic acid.

5. The invention as set forth in Claim 3, in which the preparation contains also KOH which reacts with the stearic acid to form potassium stearate.

6. The invention as set forth in any of Claims 2, 3, 4, or 5, in which only enough foaming agent or agents are included to get the preparation to be dispensed in its foam or foam-like nature, yet sufficient to provide the aforesaid time-delay breakdown of the foam or foam-like condition of the preparation while it is on the user's nail.

7. The invention as set forth in any of the preceding Claims, in which the remover preparation is packaged with a dispensing propellant or pressure means.

8. The invention as set forth in any of Claims 2 through 7, in which the remover preparation comprises ingredients as follows:

| | |
|---|---|
| Conditioner(s) or emollient(s) | about 10.2% |
| Foaming agent(s) | about 7.9% |
| Carrier(s) | about 33.7% |
| Solvent(s) | about 48.2% |

9. The invention as set forth in any of Claims 8, 10, 11, or 12, in which the 7.9% foaming agent(s) is comprised of about 7.1% stearic acid and about 0.8% KOH.

10. The invention as set forth in any of Claims 8, or 9, in which the conditioner(s) or emollient(s) comprises glycerin.

11. The invention as set forth in any of Claims 8, 9, or 10, in which the 33.7% carrier(s) is comprised of about 18.4% water and about 15.3% SDA-40 alcohol.

12. The invention as set forth in any of Claims 8, 9, 10, or 11, in which the 48.2% solvent(s) is comprised of about 30.8% acetone and about 17.4% ethyl acetate.

13. The invention as set forth in any of Claims 2 through 7, in which the remover preparation comprises ingredients as follows:

        Foaming agent
        Carrier
        Solvent

14. The invention as set forth in Claim 13, in which the carrier or carriers is/are selected from a group consisting of water, alcohol, SDA-40 alcohol, polyvinylpyrrolidone, fatty alcohols including although not limited to cetyl alcohol and oleyl and stearyl alcohol, iso-propyl alcohol, 2-propanol, carboxy polymethylene resin or carboxy vinyl polymer resin partially neutralized by triethanolamine.

15. The invention as set forth in any of Claims 13 or 14, in which the solvent or solvents is/are selected from a group consisting of acetone, ethyl acetate, other ketones including although not limited to methyl isobutyl ketone and methyl ethyl ketone, other acetates including although not limited to methyl acetate and isopropyl acetate and butyl acetate, esters, iso-propyl alcohol, 2-propanol, mono and dialkyl ethers of ethylene glycol and their derivatives, ethylene glycol monomethyl ether, N-methyl-2-pyrrolidone.

16. The invention as set forth in any of Claims 13, 14, or 15, in which the remover preparation also includes conditioner(s) and/or emollient(s) selected from a group consisting of glycerin, lanolin, peanut oil, esters including although not limited to ethyl acetate, mineral oil, fatty esters, glycols, sorbitol, fatty acid amido betaine derivatives, fatty acid amino betain derivatives, carboxy polymethylene resin or carboxy vinyl polymer resin partially neutralized by triethanolamine, and polyhydric alcohols.

17. A remover preparation for removing nail polish, in particular according to Claim 1, in which the remover preparation comprises ingredients as follows:

Carrier(s)
Solvent(s)

18. The invention as set forth in any of Claim 17, in which the carrier or carriers is/are selected from a group consisting of water, alcohol, SDA-40 alcohol, polyvinylpyrrolidone, fatty alcohols including although not limited to cetyl alcohol and oleyl and stearyl alcohol, iso-propyl alcohol, 2-propanol, carboxy polymethylene resin or carboxy vinyl polymer resin partially neutralized by triethanolamine.

19. The invention as set forth in any of Claims 17, or 18, in which the solvent or solvents is/are selected from a group consisting of acetone, ethyl acetate, other ketones including although not limited to methyl iso-butyl ketone and methyl ethyl ketone, other acetates including although not limited to methyl acetate and iso-propyl acetate and butyl acetate, esters, iso-propyl alcohol, 2-propanol, mono and dialkyl ethers of ethylene glycol and their derivatives, ethylene glycol monomethyl ether, N-methyl-2-pyrrolidone.

20. The invention as set forth in any of Claims 17, 18, or 19, in which the remover preparation also includes conditioner(s) and/or emollient(s) selected from a group consisting of glycerin, lanolin, peanut oil, esters including although not limited to ethyl acetate, mineral oil, fatty esters, glycols, Sorbitol, fatty acid amido betaine derivatives, fatty acid amino betain derivatives, carboxy plymethylene resin or carboxy vinyl polymer resin partially neutralized by triethanolamine.

21. The invention as set forth in any of Claims 17, 18, 19, or 20, in which the remover preparation comprises ingredients as follows:

| | |
|---|---|
| Conditioner(s) or emollient(s) | about 10% |
| Foaming agent(s) | about 7.8% |
| Carrier(s) | about 35% |
| Solvent(s) | about 47.2% |

22. The invention as set forth in Claim 21, in which the 10% conditioner(s) or emollient(s) comprises glycerin.

23. The invention as set forth in any of Claims 21, or 22, in which the 7.8% foaming agent(s) is comprised of about 7% stearic acid and about 0.8% KOH.

24. The invention as set forth in any of Claims 21, 22, or 23, in which the 35% carrier(s) is comprised of about 20% water and about 15% SDA-40 alcohol.

25. The invention as set forth in any of Claims 21, 22, 23, or 24, in which the 47.2% solvent(s) is comprised of acetone.

26. The invention as set forth in any of Claims 17, 18, 19, or 20, in which the remover preparation comprises ingredients as follows:

| | |
|---|---|
| Ethyl acetate | about 41.2% |
| Methyl "Cellosolve" | about 13.4% |
| IPA | about 10.3% |
| TEA "Carbopol 941" (0.2% wt.) | about 5.2% |
| Acetone | about 19.6% |
| Calcium stearate (55% water solution, by weight) | about .10.3% |

27. The invention as set forth in any of Claims 17, 18, 19, or 20, in which the remover preparation comprises ingredients as follows:

- 22 -

| | |
|---|---|
| Ethyl acetate | about 48.4% |
| Methyl "Cellosolve" | about 15.5% |
| Acetone | about 10.3% |
| TEA "Carbopol 941" | about 5.2% |
| Calcium stearate (55% water solution, by weight) | about 20.6% |

28. The invention as set forth in any of Claims 17, 18, 19, or 20, in which the remover preparation comprises ingredients as follows:

| | |
|---|---|
| Ethyl acetate | about 48.4% |
| Methyl "Cellosolve" | about 15.5% |
| Acetone | about 20.6% |
| TEA "Carbopol 941" | about 5.2% |
| Calcium stearate (55% water solution, by weight) | about 10.3% |

29. The invention as set forth in any of Claims 17, 18. 19, or 20, in which the remover preparation comprises ingredients as follows:

| | |
|---|---|
| Acetone | about 23.5% |
| Meyhyl ethyl ketone | about 47.4% |
| Water | about 23.5% |
| "Carbopol 934" | less than 1% |
| GAFAC RE-610 | about 2.6% |
| GAFAMIDE CDD-518 | about 2.6% |

30. The invention as set forth in any of Claims 17, 18, 19, or 20, in which the remover preparation comprises ingredients as follows:

|  |  |
|---|---|
| Ethylene glycol monomethyl ether, $CH_3OCH_2CH_2OH$, and/or Ethyl acetate, $CH_3COOC_2H_5$ | about 73% |
| N-methyl-2-pyrrolidone, $H_3CNCH_2CH_2CH_2CO$ | about 7.3% |
| Calcium stearate | about 3.6% |
| Coco amino betaine | about 0.7% |
| POE (20) Sorbitan monostearate | about 0.7% |
| Carboxy polymethylene | about 14.6% |

31. The invention as set forth in any of Claims 17, 18, 19, 20, or 30, in which the remover preparation is provided in the form of a gel.

32. The invention as set forth in any of Claims 17, 18, 19, 20, or 30, in which the remover preparation is provided in the form of a paste.

33. The invention as set forth in any of Claims 17, 18, 19, 20, or 30, in which the remover preparation is provided in the form of a foam.

34. The invention as set forth in any of Claims 17, 18, 19, 20, or 30, in which the remover preparation is provided in the form of a substance having a viscosity very significantly greater than that of acetone.

35. The invention as set forth in any of Claims 31, 32, 33, or 34, in which the remover substance is provided to have a significantly lower flash point than acetone.

## European Patent Office

## EUROPEAN SEARCH REPORT

| | DOCUMENTS CONSIDERED TO BE RELEVANT | | CLASSIFICATION OF THE APPLICATION (Int. Cl. ³) |
|---|---|---|---|
| Category | Citation of document with indication, where appropriate, of relevant passages | Relevant to claim | A 61 K 7/047 |
| | <u>GB - A - 975 605</u> (ALLIED CHEM. <u>CORP.</u>) <br> * Claims; page 1, line 72 - page 2, line 1; page 1, lines 37-60; page 2, line 10 - page 3, line 24 * <br><br> -- | 1,3,7, 8,10, 13,14, 15-20, 34,35 | |
| | <u>US - A - 3 131 152</u> (E. KLAUSNER) <br> * Claims; column 1, line 48 - column 3, line 2; example 4 * <br><br> -- | 1,3,7, 8,10, 12,13-20,34, 35 | TECHNICAL FIELDS SEARCHED (Int.Cl. ³) |
| | <u>GB - A - 1 306 508</u> (J.G. SPITZER) <br> * Claims; page 2, line 83 - page 6, line 121 * <br><br> -- | 1 | A 61 K 7/047 |
| | <u>GB - A - 1 384 267</u> (BRIST ..-MYERS <u>CO.</u>) <br> * Claims; page 2, line 126 - page 8, line 99 * <br><br> -- | 1 | |
| A | <u>US - A - 2 268 642</u> (H.M. CARTER) <br> * Claims; page 1, column 2, lines 35-51; page 3, column 2, lines 29-45 * <br><br> ---- | 4,6 | CATEGORY OF CITED DOCUMENTS <br> X: particularly relevant <br> A: technological background <br> O: non-written disclosure <br> P: intermediate document <br> T: theory or principle underlying the invention <br> E: conflicting application <br> D: document cited in the application <br> L: citation for other reasons |
| | The present search report has been drawn up for all claims | | &: member of the same patent family, corresponding document |

| Place of search | Date of completion of the search | Examiner |
|---|---|---|
| The Hague | 20-12-1979 | VANHECKE |

EPO Form 1503.1  06.78